(19) Europäisches Patentamt
European Patent Office
Office européen des brevets

(11) Veröffentlichungsnummer : **0 088 250**
**B1**

(12) # EUROPÄISCHE PATENTSCHRIFT

(45) Veröffentlichungstag der Patentschrift : **28.05.86**

(21) Anmeldenummer : **83101261.2**

(22) Anmeldetag : **10.02.83**

(51) Int. Cl.⁴ : **C 07 D495/04**, A 61 K 31/44 // (C07D495/04, 333:00, 221:00)

(54) **Neue basisch substituierte 4-Phenyl-4,5,6,7-tetrahydrothieno-(2,3-c)pyridine, Verfahren zu ihrer Herstellung und diese enthaltende pharmazeutische Zusammensetzungen.**

(30) Priorität : **05.03.82 DE 3207939**

(43) Veröffentlichungstag der Anmeldung : **14.09.83 Patentblatt 83/37**

(45) Bekanntmachung des Hinweises auf die Patenterteilung : **28.05.86 Patentblatt 86/22**

(84) Benannte Vertragsstaaten : **AT BE CH DE FR IT LI LU NL SE**

(56) Entgegenhaltungen :
**DE-A- 2 746 443**
**Die Akte enthält technische Angaben, die nach dem Eingang der Anmeldung eingereicht wurden und die nicht in dieser Patentschrift enthalten sind.**

(73) Patentinhaber : **BOEHRINGER INGELHEIM KG**
**D-6507 Ingelheim am Rhein (DE)**

(72) Erfinder : **Schneider, Claus, Dr.**
**Albrecht-Dürer-Strasse 19**
**D-6507 Ingelheim/Rhein (DE)**
Erfinder : **Walther, Gerhard, Dr.**
**Pfarrer-Heberer-Strasse 37**
**D-6530 Bingen/Rhein (DE)**
Erfinder : **Weber, Karl-Heinz, Dr.**
**Kaiser-Karl-Strasse 11**
**D-6535 Gau-Algesheim (DE)**
Erfinder : **Bechtel, Wolf Dietrich, Dr.**
**Mühlstrasse 3**
**D-6531 Appenheim (DE)**
Erfinder : **Böke-Kuhn, Karin, Dr.**
**Beethovenstrasse 11**
**D-6535 Gau-Algesheim (DE)**

EP 0 088 250 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue 4-Phenyl-4,5,6,7-tetrahydro-thieno [2,3-c] pyridine, Verfahren zu ihrer Herstellung und diese enthaltende pharmazeutische Zusammensetzungen.

Aus der DE-OS-2 746 443 sind strukturverwandte Verbindungen mit gleicher Wirkungsrichtung bekanntgeworden. Diese Verbindungen unterscheiden sich hauptsächlich durch den Substituenten in 2-Stellung des Moleküls von den neuen Stoffen.

In den neuen Verbindungen der allgemeinen Formel

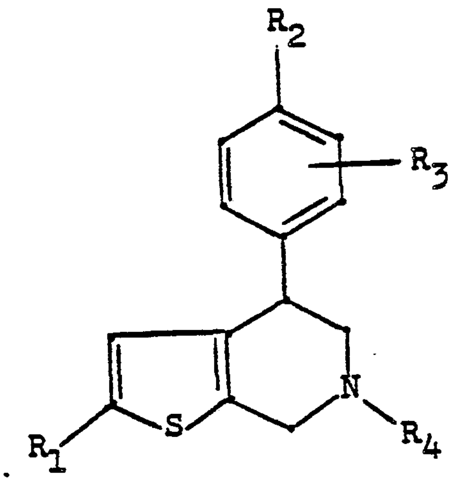

(I)

bedeuten :

$R_1$ Amino, Acetylamino,

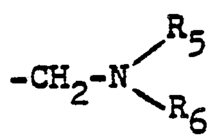

Hydroxymethyl oder Methoxymethyl,

$R_2$ und $R_3$, die gleich oder verschieden sein können, Wasserstoff, Fluor, Chlor, Brom, Trifluormethyl, Hydroxy, Methoxy, Methyl oder Ethyl,

$R_4$ einen geraden oder verzweigten Alkylrest mit 1-3 Kohlenstoffatomen und

$R_5$ und $R_6$, die gleich oder verschieden sein können, Wasserstoff, Alkylreste mit 1-4 Kohlenstoffatomen oder zusammen mit dem Stickstoffatom einen 5-, 6- oder 7-gliedrigen Ring, wobei in dem 6-Ring gegebenenfalls eine $CH_2$-Gruppe durch ein Sauerstoffatom ersetzt sein kann.

Die Erfindung umfaßt ferner die physiologisch verträglichen Säureadditionssalze der vorstehend genannten Endprodukte.

Die neuen Verbindungen können hergestellt werden, ausgehend von einem Thieno-[2,3-c]-pyridin der allgemeinen Formel

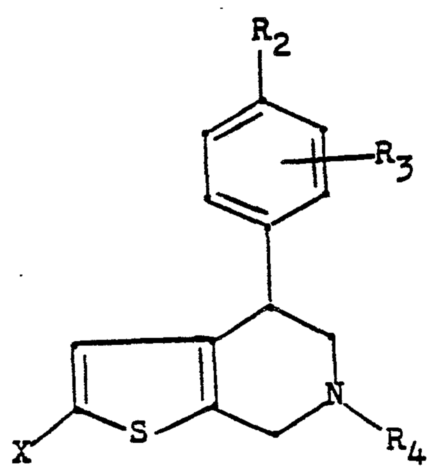

(II)

worin X einen der Reste $NO_2$, $ClCH_2$—, $BrCH_2$— oder HOC— bedeutet und die Reste $R_2$, $R_3$ und $R_4$ die oben angegebene Bedeutung haben.

Endprodukte der allgemeinen Formel I, in denen $R_1$ eine Aminogruppe bedeutet, werden erhalten

durch Reduktion der Nitrogruppe mit geeigneten Reduktionsmitteln wie Platin/Kohle oder Palladium. Das Verfahren wird in Lösungsmitteln wie Tetrahydrofuran, einem Alkohol, einen Äther oder Dioxan durchgeführt. Anstelle der vorstehend genannten Reduktionsmittel kann die Nitroverbindung auch in Eisessig gelöst und mit einer Lösung von Zinn-II-Chlorid in Salzsäure zur Aminoverbindung reduziert werden. Die Aminoverbindung kann dann gewünschtenfalls in üblicher Weise, z. B. durch Umsetzung mit Essigsäureanhydrid, an der Aminogruppe acetyliert werden.

Verbindungen der allgemeinen Formel I, in denen $R_1$ für den Rest

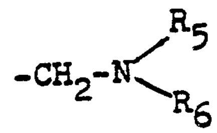

steht, erhält man, ausgehend von einer Verbindung der allgemeinen Formel II, in der X den Rest Hal—$CH_2$— bedeutet; diese Verbindung wird mit dem gewünschten Amin beziehungsweise mit Ammoniak oder einer Ammoniak liefernden Verbindung umgesetzt, entweder in einem inerten Lösungsmittel wie Äther, Dimethylformamid, Tetrahydrofuran oder einem Alkohol oder aber mit einem Überschuß des verwendeten Amins bei Temperaturen, die bevorzugt zwischen 50 und 100 °C liegen. Auch die 2-Methoxymethyl-Verbindung wird über die entsprechende 2-Halogenmethyl-Ausgangsverbindung der allgemeinen Formel II durch Umsetzung mit Natriummethylat in einem niederen Alkohol, vorzugsweise Methanol, erhalten.

Bei der Herstellung solcher Endprodukte, in denen der Rest $R_1$ eine Alkyl-amino-methyl-gruppe oder die hydroxymethylgruppe bedeutet, geht man vorzugsweise von den entsprechenden 2-Formyl-Ausgangsprodukten II aus.

Im ersten Fall wird eine Formylverbindung mit einem Überschuß eines primären Amins und mit einem Reduktionsmittel wie Raney-Nickel umgesetzt.

Die Hydroxymethyl-Verbindung erhält man durch Zugabe von Natriumborhydrid und einem geeigneten Lösungsmittel, z. B. Dioxan.

Man kann auch von einem 2-Hydroxymethyl-Endprodukt der allgemeinen Formel I zur entsprechenden 2-Methoxymethyl-Verbindung gelangen, indem man die Hydroxymethyl-Verbindung zunächst mit Thionylchlorid und anschließend mit Natriummethylat in Methanol umsetzt.

Nach den oben beschriebenen Verfahren können beispielsweise die folgenden Endprodukte, gegebenenfalls in Form ihrer Säurefadditionssalze, erhalten werden:

2-Amino-4-p-bromphenyl-6-methyl-4,5,6,7-tetrahydro-thieno [2,3-c] pyridin,
2-Amino-4-p-chlorphenyl-6-methyl-4,5,6,7-tetrahydro-thieno [2,3-c] pyridin,
2-Acetylamino-4-p-bromphenyl-6-methyl-4,5,6,7-tetrahydro-thieno [2,3-c] pyridin,
2-Acetylamino-4-p-chlorphenyl-6-methyl-4,5,6,7-tetrahydro-thieno [2,3-c] pyridin,
2-Aminomethyl-4-p-chlorphenyl-6-methyl-4,5,6,7-tetrahydro-thieno [2,3-c] pyridin,
2-Methyl-aminomethyl-4-p-methoxyphenyl-6-methyl-4,5,6,7-tetrahydro-thieno [2,3-c] pyridin,
2-tert.-Butylaminomethyl-4-p-chlorphenyl-6-methyl-4,4,5,6,7-tetrahydro-thieno [2,3-c] pyridin,
2-Äthylaminomethyl-4-p-methoxyphenyl-6-methyl-4,5,6,7-tetrahydro-thieno [2,3-c] pyridin,
2-Diäthylaminomethyl-4-p-bromphenyl-6-methyl-4,5,6,7-tetrahydro-thieno [2,3-c] pyridin,
2-Diäthylaminomethyl-4-p-chlorphenyl-6-methyl-4,5,6,7-tetrahydro-thieno [2,3-c] pyridin,
2-Morpholinomethyl-4-p-bromphenyl-6-methyl-4,5,6,7-tetrahydro-thieno [2,3-c] pyridin,
2-Morpholinomethyl-4-p-chlorphenyl-6-methyl-4,5,6,7-tetrahydro-thieno [2,3-c] pyridin,
2-Morpholinomethyl-4-p-methoxyphenyl-6-methyl-4,5,6,7-tetrahydro-thieno [2,3-c] pyridin,
2-Pyrrolidinomethyl-4-p-chlorphenyl-6-methyl-4,5,6,7-tetrahydro-thieno [2,3-c] pyridin,
2-Pyrrolidinomethyl-4-p-bromphenyl-6-methyl-4,5,6,7-tetrahydro-thieno [2,3-c] pyridin,
2-Pyrrolidinomethyl-4-p-methoxyphenyl-6-methyl-4,5,6,7-tetrahydro-thieno [2,3] pyridin,
2-Hydroxymethyl-4-p-tolyl-6-methyl-4,5,6,7-tetrahydro-thieno [2,3-c] pyridin,
2-Hydroxumethyl-4-p-methoxyphenyl-6-methyl-4,5,6,7-tetrahydro-thieno [2,3-c] pyridin,
2-Hydroxymethyl-4-p-fluorphenyl-6-methyl-4,5,6,7-tetrahydro-thieno [2,3-c] pyridin,
2-Methoxymethyl-4-p-bromphenyl-6-methyl-4,5,6,7-tetrahydro-thieno [2,3-c] pyridin,
2-Methoxymethyl-4-p-chlorphenyl-6-methyl-4,5,6,7-tetrahydro-thieno [2,3-c] pyridin,
2-Hydroxymethyl-4-(3,4-dimethoxyphenyl)-6-methyl-4,5,6,7-tetrahydro-thieno [2,3-c] pyridin,
2-Hydroxymethyl-4-(3,4-dihydroxyphenyl)-6-methyl-4,5,6,7-tetrahydro-thieno [2,3-c] pyridin,
2-Hexamethyleniminomethyl-4-(3,4-dichlorphenyl-6-methyl-4,5,6,7-tetrahydro-thieno [2,3-c] pyridin.

Ausgangsverbindungen der allgemeinen Formel II lassen sich nach an sich bekannten Verfahren erhalten:

Eine Nitrogruppe läßt sich in 2-Stellung des Moleküls durch Nitrierung der an dieser Stelle unsubstituierten Verbindung mit Trifluoressigsäure und rauchender Salpetersäure unter Kühlung einführen.

Verbindungen, in denen X eine Halogenmethylgruppe bedeutet, erhält man beispielsweise durch Umsetzung der entsprechenden 2-Methyl-Verbindung mit einem N-Halogensuccinimid in Gegenwart eines Radikalstarters, z. B. Azobis-isobutyronitril, unter Rückfluß.

2-Formyl-Ausgangsverbindungen lassen sich erhalten, indem man die entsprechende 2-Halogen-Verbindung in absolutem Äther bei Raumtemperatur mit Butyl-Lithium versetzt und anschließend unter Kühlung Dimethylformamid zugibt.

Es ist möglich, aber nicht unbedingt erforderlich, die so hergestellten Ausgangsstoffe der allgemeinen Formel II zu isolieren, sie können auch *in situ* für die Umsetzung zu den gewünschten Endprodukten eingesetzt werden.

Die Endprodukte der allgemeinen Formel I können gewünschtenfalls nach üblichen Methoden in ihre physiologisch unbedenklichen Säureadditionssalze überführt werden.

Als Säuren eignen sich hierfür sowohl anorganische Säuren wie Halogenwasserstoffsäuren, Schwefelsäure, Phosphorsäure und Aminosulfonsäure, als auch organische Säuren wie Ameinsensäure, Essigsäure, Propionsäure, Milchsäure, Glykolsäure, Glukonsäure, Maleinsäure, Bernsteinsäure, Weinsäure, Benzoesäure, Salicylsäure, Zitronensäure, Ascorbinsäure, p-Toluolsulfonsäure oder Oxyäthansulfonsäure.

Die neuen Stoffe der allgemeinen Formel I und ihre Säureadditionssalze stellen wertvolle Pharmazeutika dar. In einigen spezifischen Tests hat sich herausgestellt, daß sie starke antidepressive Eigenschaften besitzen und insbesondere eine thymoleptische und zentralanregende Wirkung ausüben.

Ein Test zur Bestimmung der antidepressiven Wirkung ist der Reserpin-Antagonismus, die Aufhebung des durch Reserpin verursachten hypothermischen Effektes. Der Versuch wird an Mäusen durchgeführt, pro Dosis werden 5 Tiere verwendet. 17 Stunden nach einer i.p.-Gabe von 2 mg/kg Reserpin wird bei einer Raumtemperatur von 19 °C die Körpertemperatur peripher gemessen. Danach wird die Testsubstanz oral gegeben und die Körpertemperatur nach Ablauf von 1, 3, 5 und 7 Stunden gemessen. Für jeden Zeitpunkt der Messung wird eine mittlere Wirkdosis ($ED_{50}$) bestimmt. Dies ist diejenige Dosis, bei welcher die Körpertemperatur der mit Reserpin behandelten Tiere der Normaltemperatur der unbehandelten Kontrolltiere um 50 % angenähert ist.

Die folgende Tabelle zeigt die Ergebnisse :

(Siehe Tabelle Seite 5 f.)

| Verbindung | Reserpin-Antagonismus nach (mg/kg) | | | | $LD_{50}$ Maus oral |
|---|---|---|---|---|---|
| | 1 | 3 | 5 | 7 h | |
| 2-Hydroxymethyl-6-methyl-4-p-tolyl-4,5,6,7-tetrahydro-thieno[2,3-c]pyridin-hydrochlorid | 2,5 | 0,24 | 2,5 | > 10 | ~ 740 |
| 2-Morpholinomethyl-4-p-bromphenyl-6-methyl-4,5,6,7-tetrahydro-thieno[2,3-c]pyridin-dimaleat | 22 | 1,7 | 1,7 | 10,0 | > 640 |
| 2-Morpholinomethyl-4-p-chlorphenyl-6-methyl-4,5,6,7-tetrahydro-thieno[2,3-c]pyridin-dimaleat | > 40 | 2,6 | 12,0 | 2,5 | > 640 |

Beispiel 1

2-Acetylamino-4-p-bromphenyl-6-methyl-4,5,6,7-tetrahydro-thieno [2,3-c] pyridin

4 g (0,01 Mol) 4-p-Bromphenyl-6-methyl-2-nitro-4,5,6,7-tetrahydro-thieno [2,3-c] pyridin werden in 20 ml Eisessig suspendiert und mit 20 g Zinn-II-Chlorid in 40 ml konzentrierter Salzsäure versetzt. Nach beendeter Reaktion wird mit Eis versetzt, die Lösung alkalisch gestellt und mit Äther extrahiert. Nach Abdampfen des Lösungsmittels wird der Rückstand mit 25 ml Acetanhydrid versetzt und kurze Zeit auf 50 °C erwärmt. Anschließend wird Wasser zugegeben, alkalisch gemacht und mit Essigester extrahiert. Nach dem Einengen des Lösungsmittels werden die angefallenen Kristalle abgesaugt.

Ausbeute : 2 g (55 % d. Th.) ; Fp. 218-220 °C (Zers.)

Die Ausgangsverbindung wurde auf folgendem Weg erhalten :

Zu 14,5 g (0,047 Mol) 4-p-Bromphenyl-6-methyl-tetrahydro-thieno [2,3-c] pyridin in 100 ml Trifluoressigsäure werden unter Kühlung 25 ml rauchende Salpetersäure so zugetropft, daß die Temperatur 20 °C nicht übersteigt. Nach beendeter Zugabe wird noch 30 Minuten gerührt. Die Reaktionsmischung wird auf Eis gegossen, mit konzentriertem Ammoniak bis zur alkalischen Reaktion versetzt und mit Methylenchlorid extrahiert. Nach dem Abdampfen des Lösungsmittels kristallisiert aus alkoholischer Salzsäure das Hydrochlorid des 4-p-Bromphenyl-6-methyl-2-nitro-4,5,6,7-tetrahydro [2,3-c] pyridins.

Ausbeute : 12 g (66 % d. Th.) ; Fp. 205-207 °C (Zers.)

Beispiel 2

2-Amino-4-p-chlorphenyl-6-methyl-4,5,6,7-tetrahydro-thieno [2,3-c] pyridin

7 g (0,002 3 Mol) 4-p-Chlorphenyl-6-methyl-2-nitro-tetrahydro-thieno [2,3-c] pyridin werden in 70 ml Tetrahydrofuran/Alkohol (1 : 1) mehrere Stunden mit 0,7 g Platin/Kohle bei 5 bar Wasserstoffdruck behandelt. Nach Abfiltrieren des Katalysators erhält man durch Zugabe von alkoholischer Maleinsäure das Maleat der Titelverbindung.

Ausbeute : 5,4 g (60 % d. Th.) ; Fp. 165-166 °C (Zers.)

Beispiel 3

2-Morpholinomethyl-4-p-bromphenyl-6-methyl-4,5,6,7-tetrahydro-thieno [2,3-c] pyridin

6,46 g (0,02 Mol) 4-p-Bromphenyl-2,6-dimethyl-tetrahydro-thieno [2,3-c] pyridin werden in 50 ml Tetrachlorkohlenstoff mit 2,6 g N-Bromsuccinimid und 0,1 g Azo-bis-isobutyronitril mehrere Stunden zum Sieden erhitzt. Danach wird das entstandene Succinimid abgesaugt und das Lösungsmittel abgedampft. Der Rückstand wird langsam mit überschüssigem Morpholin versetzt und 10 Minuten bei 50 °C gerührt. Die Titelverbindung wird aus der wäßrigen Phase mit Äther extrahiert. Sie kristallisiert aus Aceton/Maleinsäure als Dimaleat.

Ausbeute : 5,85 g (46 % d. Th.) ; Fp. 155-156 °C (Zers.)

Beispiel 4

2-Methoxymethyl-4-p-chlorphenyl-6-methyl-4,5,6,7-tetrahydro-thieno [2,3-c] pyridin

2,79 g (0,01 Mol) 4-p-Chlorphenyl-2,6-dimethyl-tetrahydro-thieno [2,3-c] pyridin werden in 50 ml Tetrachlorkohlenstoff gelöst und mit 1,8 g N-Bromsuccinimid und 0,1 g Azobis-isobutyronitril mehrere Stunden zum Sieden erhitzt. Das entstandene Succinimid wird abgesaugt und das Lösungsmittel am Rotationsverdampfer entfernt. Der Rückstand wird mit 0,01 Mol Natrium-Methylat in Methanol versetzt und kurze Zeit zum Sieden erhitzt. Anschließen wird mit Wasser versetzt und mit Äther extrahiert. Durch Zugabe von ätherischer Salzsäure kristallisiert das Hydrochlorid der Titelverbindung.

Ausbeute : 1,74 g (53 % d. Th.) ; Fp. 238-239 °C (Zers.)

Beispiel 5

2-Hydroxymethyl-6-methyl-4-p-tolyl-4,5,6,7-tetrahydro-thieno [2,3-c] pyridin

22 g 2-Formyl-6-methyl-4-p-tolyl-4,5,6,7-tetrahydro-thieno [2,3-c] pyridin werden in 100 ml Dioxan gelöst und mit 4 g Natriumborhydrid in 50 ml Wasser langsam vermischt. Nach beendeter Reaktion wird überschüssiges Natriumborhydrid mit Salzsäure zerstört. Aus der ammoniakalischen Lösung wird die Titelverbindung mit Äther extrahiert als Hydrochlorid gefällt.

Ausbeute : 12,3 g (58 % d. Th.) ; Fp. 238-239 °C (Äthanol)

Die Ausgangsverbindung wurde wie folgt erhalten :

Zu 32,3 g (0,1 Mol) 2-Brom-6-methyl-4-p-tolyl-tetrahydro-thieno [2,3-c] pyridin in 230 ml absolutem

Äther werden bei —30 °C 0,15 Mol Butyl-Lithium in n-Hexan zugetropft und bei Raumtemperatur 2 Stunden gerührt. Danach werden bei —30 °C 20 ml Dimethylformamid langsam zugegeben. Nach beendeter Reaktion wird zuerst mit Eis, dann mit 2 n Salzsäure versetzt. Mit Ammoniak wird neutralisiert und mit Essigester extrahiert.

Ausbeute an 2-Formyl-6-methyl-4-p-tolyl-4,5,6,7-tetrahydro-thieno [2,3-c] pyridin : 22 g (80 % d. Th.) ; hellgelbes Öl.

Beispiel 6

2-Methylaminomethyl-4-p-methoxyphenyl-6-methyl-4,5,6,7-tetrahydro-thieno [2,3-c] pyridin

2,87 g (0,01 Mol) 2-Formyl-4-p-methoxyphenyl-6-methyl-tetrahydro-thieno [2,3-c] pyridin (hergestellt analog Beispiel 5 ; hellgelbes Öl) in 70 ml Tetrahydrofuran werden mit einem Überschuß von Methylamin und Raney-Nickel unter 5 bar Wasserstoffdruck mehrere Stunden bei 60 °C gehalten. Nach Absaugen des Katalysators wird die Titelverbindung durch Zugabe alkoholischer Maleinsäurelösung als Dimaleat gefällt.

Ausbeute : 3,4 g (65 % d. Th.) ; Fp. 143-144 °C

Beispiel 7

2-Morpholinomethyl-4-p-chlorphenyl-6-methyl-4,5,6,7-tetrahydro-thieno [2,3-c] pyridin

4,54 g (0,02 Mol) 2-Hydroxymethyl-4-p-chlorphenyl-6-methyl-4,5,6,7-tetrahydro-thieno [2,3-c] pyridin werden in 30 ml Äthylenchlorid mit überschüssigem Thionylchlorid erwärmt. Nach beendeter Reaktion wird unter Vakuum eingeengt, langsam mit überschüssigem Morpholin versetzt und 10 Minuten bei 50 °C gerührt. Die Titelverbindung wird aus der wäßrigen Phase mit Äther extrahiert. Sie kristallisiert aus Aceton/Maleinsäure als Dimaleat.

Ausbeute : 6,2 g (52 % d. Th.) ; Fp. 162-163 °C (Zers.)

Beispiel 8

2-Methoxymethyl-4-p-chlorphenyl-6-methyl-4,5,6,7-tetrahydro-thieno [2,3-c] pyridin

4,54 g (0,02 Mol) der nach B ispiel 7 erhaltenen 2-Hydroxymethyl-Verbindung werden, wie in Beispiel 7 beschrieben, mit Thionylchlorid behandelt. Nach beendeter Reaktion wird mit 0,02 Mol Natrium-Methylat in Methanol versetzt und kurze Zeit zum Siedem erhitzt. Anschließend wird Wasser zugegeben und mit Äther extrahiert. Die Titelverbindung wird als Hydrochlorid gefällt.

Ausbeute : 4,2 g (64 % d. Th.) ; Fp. 238-239 °C (Zers.)

Nach den vorstehend beschriebenen Verfahren wurden folgende in der Tabelle aufgeführten Verbindungen erhalten :

(Siehe Tabelle Seite 8 f.)

| Beispiel | $R_1$ | $R_2$ | $R_3$ | $R_4$ | Fp. °C | Salz |
|---|---|---|---|---|---|---|
| 9 | $-NH_2$ | Br | H | $CH_3$ | 145-147 (Zers.) | Maleinat |
| 10 | $-NH-CO-CH_3$ | Cl | H | $CH_3$ | 227-229 (Zers.) | |
| 11 | $H_2N-CH_2-$ | Cl | H | $CH_3$ | 186-187 (Zers.) | Dimaleinat |
| 12 | $t-Bu-NH-CH_2-$ | Cl | H | $CH_3$ | 215-216 (Zers.) | " |
| 13 | $(Et)_2N-CH_2-$ | Br | H | $CH_3$ | 117-118 (Zers.) | " |
| 14 | $(Et)_2N-CH_2-$ | Cl | H | $CH_3$ | 75 (Zers.) | " |
| 15 | 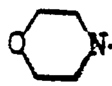N-$CH_2$- | $OCH_3$ | H | $CH_3$ | 156-157 (Zers.) | " |
| 16 | 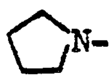N-$CH_2$- | Cl | H | $CH_3$ | 172-173 (Zers.) | " |
| 17 | 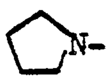N-$CH_2$- | Br | H | $CH_3$ | 169-170 (Zers.) | " |
| 18 | 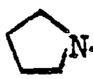N-$CH_2$- | $OCH_3$ | H | $CH_3$ | 178-179 (Zers.) | " |
| 19 | $CH_3O-CH_2-$ | Br | H | $CH_3$ | 167-168 (Zers.) | Methansulfonat |
| 20 | $HO-CH_2-$ | $OCH_3$ | H | $CH_3$ | 221-222 | Chlorid |
| 21 | $C_2H_5-NHCH_2-$ | $OCH_3$ | H | $CH_3$ | 147-148 | Dimaleinat |
| 22 | 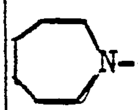N-$CH_2$- | Cl | m-Cl | $CH_3$ | 172-173 | Chlorid |
| 23 | $HO-CH_2-$ | H | H | $CH_3$ | 220-221 | " |
| 24 | $HO-CH_2-$ | F | H | $CH_3$ | 210-211 | " |

Formulierungsbeispiele

a) Dragees

| | |
|---|---|
| 1 Drageekern enthält : | |
| Wirkstoff gemäß der Erfindung | 25,0 mg |
| Milchzucker | 50,0 mg |
| Maisstärke | 22,0 mg |
| Gelatine | 2,0 mg |
| Magnesiumstearat | 1,0 mg |
| | 100,0 mg |

Herstellung

Die Mischung der Wirksubstanz mit Milchzucker und Maisstärke wird mit einer 10 %igen wäßrigen Gelatinelösung durch ein Sieb mit 1 mm Maschenweite granuliert, bei 40 °C getrocknet und nochmals durch ein Sieb getrieben. Das so erhaltene Granulat wird mit Magnesiumstearat gemischt und verpreßt. Die so erhaltenen Kerne werden in üblicher Weise mit einer Hülle überzogen, die mit Hilfe einer wäßrigen Suspension von Zucker, Titandioxyd, Talkum und Gummi arabicum aufgebracht ird. Die fertigen Dragees werden mit Bienenwachs poliert. Dragee-Endgewicht : 200 mg.

b) Tabletten

| | |
|---|---|
| Wirkstoff gemäß der Erfindung | 10,0 mg |
| Milchzucker | 40,0 mg |
| Maisstärke | 44,0 mg |
| Lösliche Stärke | 5,0 mg |
| Magnesiumstearat | 1,0 mg |
| | 100,0 mg |

Herstellung

Wirkstoff und Magnesiumstearat werden mit einer wäßrigen Lösung der löslichen Stärke granuliert, das Granulat getrocknet und inning mit Milchzucker und Maisstärke vermischt. Das Gemisch wird sodann zu Tabletten von 100 mg Gewicht verpreßt, die je 10 mg Wirkstoff enthalten.

c) Suppositorien

| | |
|---|---|
| 1 Zäpfchen enthält : | |
| Wirkstoff gemäß der Erfindung | 10,0 mg |
| Zäpfchenmasse | 1 690,0 mg |

Herstellung

Die feingepulverte Substanz wird mit Hilfe eines Eintauch-Homogenisators in die geschmolzene und auf 40 °C abgekühlte Zäpfchenmasse eingerührt. Die Masse wird bei 35 °C in leicht vorgekühlte Formen gegossen.

d) Ampullen (Injektionslösungen)

| | |
|---|---|
| Zusammensetzung : | |
| Wirkstoff gemäß der Erfindung | 5,0 Gew.-Teile |
| Natriumpyrosulfit | 1,0 Gew.-Teile |
| Dinatriumsalz der Äthylendiamintetraessigsäure | 0,5 Gew.-Teile |
| Natriumchlorid | 8,5 Gew.-Teile |
| doppelt destilliertes Wasser ad | 1 000,0 Gew.-Teile |

Herstellung

Der Wirkstoff und die Hilfsstoffe werden in einer ausreichenden Menge Wasser gelöst und mit der notwendigen Menge Wasser auf die gewünschte Konzentration gebracht. Die Lösung wird filtriert und unter aseptischen Bedingungen in 1 ml Ampullen abgefüllt. Zuletzt werden die Ampullen sterilisiert und verschlossen. Jede Ampulle enthält 5,0 mg Wirkstoff.

**Patentansprüche**

1. Neue substituierte 4-Phenyl-4,5,6,7-tetrahydro-thieno [2,3-c] pyridine der allgemeinen Formel

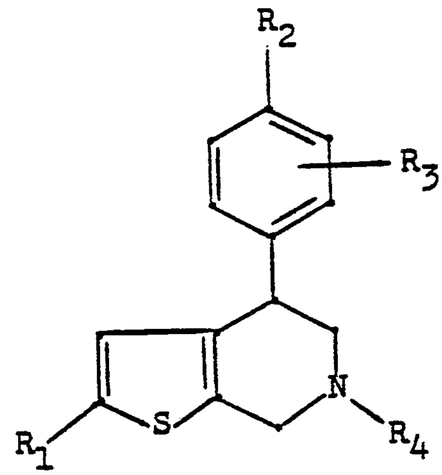

(I)

worin

$R_1$ Amino, Acetylamino,

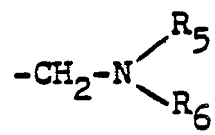

Hydroxymethyl oder Methoxymethyl,

$R_2$ und $R_3$, die gleich oder verschieden sein können, Wasserstoff, Fluor, Chlor, Brom, Trifluormethyl, Hydroxy, Methoxy, Methyl oder Ethyl,

$R_4$ einen geraden oder verzweigten Alkylrest mit 1-3 Kohlenstoffatomen und

$R_5$ und $R_6$, die gleich oder verschieden sein können, Wasserstoff, Alkylreste mit 1-4 Kohlenstoffatomen bedeuten oder zusammen mit dem Stickstoffatom einen 5-, 6- oder 7-gliedrigen Ring bilden, wobei in dem 6-Ring gegebenenfalls eine $CH_2$-Gruppe durch ein Sauerstoffatom ersetzt sein kann sowie deren physiologisch verträgliche Säureadditionssalze.

2. Neue substituierte 4-Phenyl-4,5,6,7-tetrahydro-thieno [2,3-c] pyridine der allgemeinen Formel I gemäß Anspruch 1, worin $R_1$ die Hydroxymethyl- oder die Morpholinomethylgruppe bedeutet und die Reste $R_2$, $R_3$ und $R_4$ die in Anspruch 1 angegebene Bedeutung besitzen.

3. 2-Hydroxymethyl-4-p-tolyl-6-methyl-tetrahydro-thieno [2,3-c] pyridin und dessen physiologisch verträgliche Säureadditionssalze.

4. 2-Morpholinomethyl-4-p-bromphenyl-6-methyl-tetra-hydro-thieno [2,3-c] pyridin und dessen physiologisch verträgliche Säureadditionssalze.

5. 2-Morpholinomethyl-4-p-chlorphenyl-6-methyl-tetrahydro-thieno [2,3-c] pyridin und dessen physiologisch verträgliche Säureadditionssalze.

6. 2-Morpholinomethyl-4-p-methoxyphenyl-6-methyl-tetrahydro-thieno [2,3-c] pyridin und dessen physiologisch verträgliche Säureadditionssalze.

7. Verfahren zur Herstellung neuer substituierter 4-Phenyl-4,5,6,7-tetrahydro-thieno [2,3-c] pyridine der allgemeinen Formel

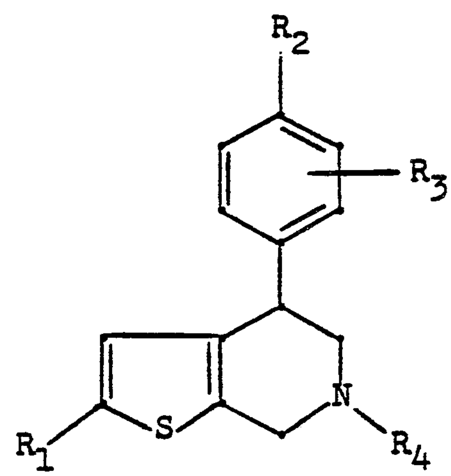

(I)

worin

$R_1$ Amino, Acetylamino,

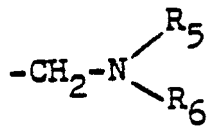

Hydroxymethyl oder Methoxymethyl,

$R_2$ und $R_3$, die gleich oder verschieden sein können, Wasserstoff, Fluor, Chlor, Brom, Trifluormethyl, Hydroxy, Methoxy, Methyl oder Ethyl,

$R_4$ einen geraden oder verzweigten Alkylrest mit 1-3 Kohlenstoffatomen und

$R_5$ und $R_6$, die gleich oder verschieden sein können, Wasserstoff, Alkylreste mit 1-4 Kohlenstoffatomen bedeuten oder zusammen mit dem Stickstoffatom einen 5-, 6- oder 7-gliedrigen Ring bilden, wobei in dem 6-Ring gegebenenfalls eine $CH_2$-Gruppe durch ein Sauerstoffatom ersetzt sein kann sowie deren physiologisch verträgliche Säureadditionssalze, dadurch gekennzeichnet, daß man in einer Verbindung der allgemeinen Formel

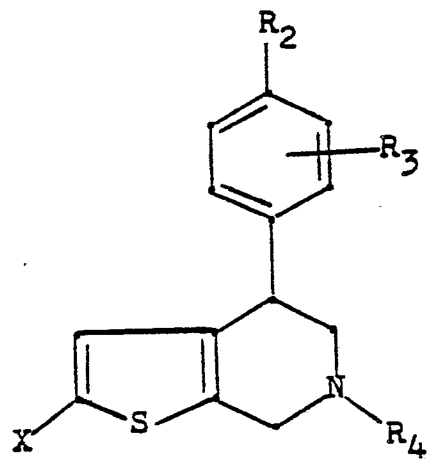

(II)

worin die Reste $R_2$, $R_3$ und $R_4$ die oben angegebene Bedeutung haben und X einen der Reste $NO_2$, $ClCH_2$—, $BrCH_2$— oder HOC— bedeutet,

a) zur Herstellung von Endprodukten, in denen $R_1$ die Aminogruppe oder die Acetylaminogruppe bedeutet, die Nitrogruppe einer Ausgangsverbindung der allgemeinen Formel II, in der X $NO_2$ bedeutet, mit geeigneten Reduktionsmitteln reduziert und die so erhaltene Aminoverbindung gewünschtenfalls in üblicher Weise acetyliert, oder daß man

b) zur Herstellung von in 2-Stellung durch den Rest

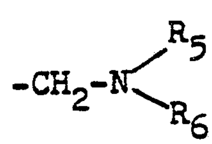

beziehungsweise die Methoxymethylgruppe substituierten Endprodukten eine Ausgangsverbindung der allgemeinen Formel II, in der X einen Halogenmethylrest bedeutet, mit dem gewünschten Amin beziehungsweise mit Ammoniak oder einer Ammoniak liefernden Verbindung beziehungsweise mit Natriummethylat umsetzt, oder daß man

c) zur Herstellung solcher Endprodukte, in denen $R_1$ einen Alkylaminomethylrest oder die Hydroxymethylgruppe bedeutet, eine Ausgangsverbindung der allgemeinen Formel II, in der X den Formylrest bedeutet, entweder mit einem primären Amin und einem geeigneten Reduktionsmittel oder mit Natriumborhydrid in an sich bekannter Weise umsetzt,

und daß man ein so erhaltenes Endprodukt der allgemeinen Formel I auf übliche Weise in ein physiologisch unbedenkliches Säureadditionssalz überführt.

8. Verfahren nach Anspruch 7, dadurch gekennzeichnet, daß man ein 2-Hydroxymethyl-thieno [2,3-c] pyridin der allgemeinen Formel I ($R_1 = HOCH_2$—) mit Thionylchlorid und anschließend mit Natriummethylat in die entsprechende 2-Methoxymethylverbindung überführt.

9. Pharmazeutische Zusammensetzungen, enthaltend als Wirkstoff eine oder mehrere Verbindungen der allgemeinen Formel I in Kombination mit üblichen Hilfs- und/oder Trägerstoffen.

**Claims**

1. New substituted 4-phenyl-4,5,6,7-tetrahydro-thieno [2,3-c] pyridines of general formula

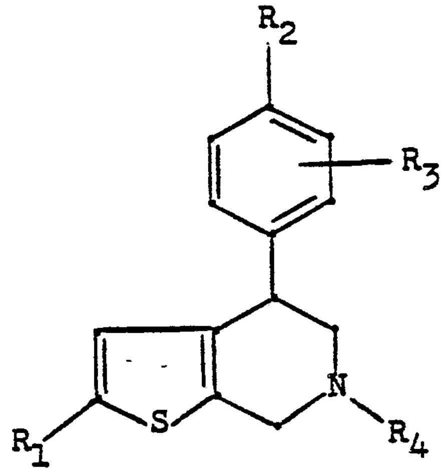

(I)

wherein

$R_1$ represents amino, acetylamino,

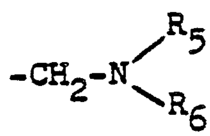

hydroxymethyl or methoxymethyl,

$R_2$ and $R_3$, which may be the same or different, represent hydrogen, fluorine, chlorine, bromine, trifluoromethyl, hydroxy, methoxy, methyl or ethyl,

$R_4$ represents a straight-chained or branched alkyl group with 1 to 3 carbon atoms and

$R_5$ and $R_6$, which may be the same or different, represent hydrogen, alkyl groups with 1 to 4 carbon atoms or, together with the nitrogen atom, represent a 5-, 6- or 7-membered ring, whilst in the 6-membered ring any $CH_2$ group present may be substituted by an oxygen atom, and the physiologically acceptable acid addition salts thereof.

2. New substituted 4-phenyl-4,5,6,7-tetrahydro-thieno [2,3-c] pyridines of general formula I as claimed in claim 1, wherein $R_1$ represents the hydroxymethyl or morpholinomethyl group and the groups $R_2$, $R_3$ and $R_4$ have the meanings given in claim 1.

3. 2-Hydroxymethyl-4-p-tolyl-6-methyl-tetrahydro-thieno [2,3-c] pyridine and the physiologically acceptable acid addition salts thereof.

4. 2-Morpholinomethyl-4-p-bromophenyl-6-methyl-tetrahydro-thieno [2,3-c] pyridine and the physiologically acceptable acid addition salts thereof.

5. 2-Morpholinomethyl-4-p-chlorophenyl-6-methyl-tetrahydro-thieno [2,3-c] pyridine and the physiologically acceptable acid addition salts thereof.

6. 2-Morpholinomethyl-4-p-methoxyphenyl-6-methyl-tetrahydro-thieno [2,3-c] pyridine and the physiologically acceptable acid addition salts thereof.

7. Process for preparing new substituted 4-phenyl-4,5,6,7-tetrahydro-thieno [2,3-c] pyridines of general formula

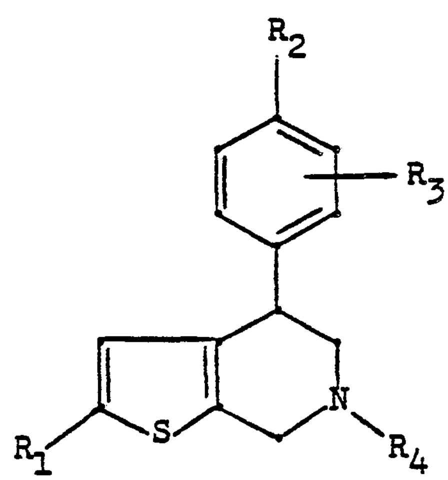

(I)

wherein

$R_1$ represents amino, acetylamino,

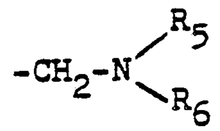

hydroxymethyl or methoxymethyl,

$R_2$ and $R_3$, which may be the same or different, represent hydrogen, fluorine, chlorine, bromine, trifluoromethyl, hydroxy, methoxy, methyl or ethyl,

$R_4$ represents a straight-chained or branched alkyl group with 1 to 3 carbon atoms and

$R_5$ and $R_6$, which may be the same or different, represent hydrogen, alkyl groups with 1 to 4 carbon atoms or, together with the nitrogen atom, represent a 5-, 6- or 7-membered ring, whilst in the 6-membered ring any $CH_2$ group present may be substituted by an oxygen atom, and the physiologically acceptable acid addition salts thereof, characterised in that in a compound of general formula

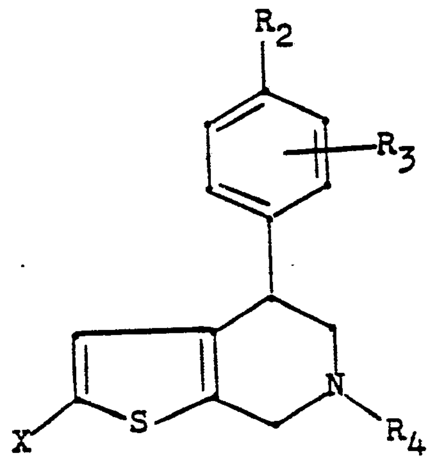

(II)

wherein the groups $R_2$, $R_3$ and $R_4$ are as hereinbefore defined and X represents one of the groups $NO_2$, $ClCH_2$—, $BrCH_2$— or HOC—,

a) in order to prepare end products wherein $R_1$ represents the amino group or the acetylamino group, the nitro group of a starting compound of general formula II wherein X represents $NO_2$ is reduced with suitable reducing agents and the amino compound thus obtained is optionally acetylated in the usual way, or

b) in order to prepare end products which are substituted in the 2-position by the group

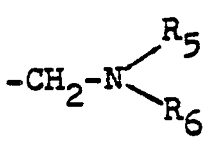

or by the methoxymethyl group, a starting compound of general formula II wherein X represents a halomethyl group is reacted with the desired amine or with ammonia or an ammonia-producing compound or with sodium methoxide, or

c) in order to prepare end products wherein $R_1$ represents an alkylaminomethyl group or the hydroxymethyl group, a starting compound of general formula II wherein X represents the formyl group is reacted either with a primary amine and a suitable reducing agent or with sodium borohydride in a manner known per se, and an end product of general formula I thus obtained is converted into a physiologically acceptable acid addition salt thereof in the conventional manner.

8. Process as claimed in claim 7, characterised in that a 2-hydroxymethyl-thieno [2,3-c] pyridine of general formula I ($R_1 = HOCH_2$) is reacted with thionyl chloride and subsequently with sodium methoxide to convert it into the corresponding 2-methoxymethyl compound.

9. Pharmaceutical compositions containing as active substance one or more compounds of general formula I together with conventional excipients and/or carriers.

**Revendications**

1. Nouvelles 4-phényl-4,5,6,7-tétrahydro-thiéno [2,3-c] pyridines substituées de formule générale

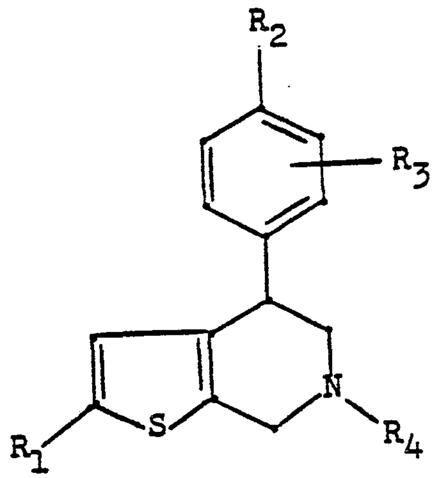

(I)

dans laquelle

$R_1$ représente amino, acétylamino,

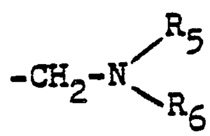

hydroxyméthyle ou méthoxyméthyle,

$R_2$ et $R_3$ qui peuvent être identiques ou différents, représentent hydrogène, fluor, chlore, brome, trifluorométhyle, hydroxy, méthoxy, méthyle ou éthyle,

$R_4$ représente un radical alcoyle rectiligne ou ramifié avec 1-3 atomes de carbone et

$R_5$ et $R_6$, qui peuvent être identiques ou différents, représentent hydrogène, des radicaux alcoyle avec 1-4 atomes de carbone ou forment ensemble avec l'atome d'azote un cycle à 5, 6 ou 7 membres, dans le cycle à 6 membres, un groupe $CH_2$ pouvant éventuellement être remplacé par un atome d'oxygène, ainsi que leurs sels d'addition d'acides physiologiquement supportables.

2. Nouvelles 4-phényl-4,5,6,7-tétrahydro-thiéno [2,3-c] pyridines substituées de formule générale I selon la revendication 1, dans laquelle $R_1$ représente le groupe hydroxyméthyle ou le groupe morpholinométhyle et les radicaux $R_2$, $R_3$ et $R_4$ possèdent la signification indiquée à la revendication 1.

3. La 2-hydroxyméthyl-4-p-tolyl-6-méthyl-tétrahydro-thiéno [2,3-c] pyridine et ses sels d'addition d'acides physiologiquement supportables.

4. La 2-morpholinométhyl-4-bromophényl-6-méthyl-tétrahydro-thiéno [2,3-c] pyridine et ses sels d'addition d'acides physiologiquement supportables.

5. La 2-morpholinométhyl-4-p-chlorophényl-6-méthyl-tétrahydro-thiéno [2,3-c] pyridine et ses sels d'addition d'acides physiologiquement supportables.

6. La 2-morpholinométhyl-4-p-méthoxyphényl-6-méthyl-tétrahydro-thiéno [2,3-c] pyridine et ses sels d'addition d'acides physiologiquement supportables.

7. Procédé pour la préparation de nouvelles 4-phényl-4,5,6,7-tétrahydro-thiéno [2,3-c] pyridines substituées de formule générale

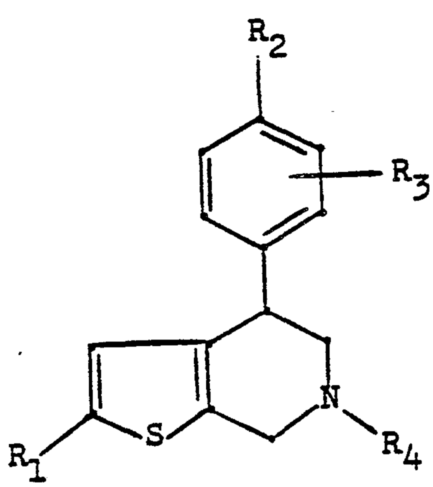

(I)

dans laquelle

$R_1$ représente amino, acétylamino,

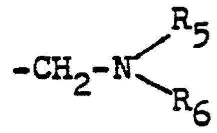

hydroxyméthyle ou méthoxyméthyle,

$R_2$ et $R_3$ qui peuvent être identiques ou différents, représentent hydrogène, fluor, chlore, brome, trifluorométhyle, hydroxy, méthoxy, méthyle ou éthyle,

$R_4$ représente un radical alcoyle rectiligne ou ramifié avec 1-3 atomes de carbone et

$R_5$ et $R_6$, qui peuvent être identiques ou différents, représentent hydrogène, des radicaux alcoyle avec 1-4 atomes de carbone ou forment ensemble avec l'atome d'azote un cycle à 5, 6 ou 7 membres, dans le cycle à 6 membres, un groupe $CH_2$ pouvant éventuellement être remplacé par un atome d'oxygène, ainsi que de leurs sels d'addition d'acides physiologiquement supportables, caractérisé en ce que dans un composé de formule générale

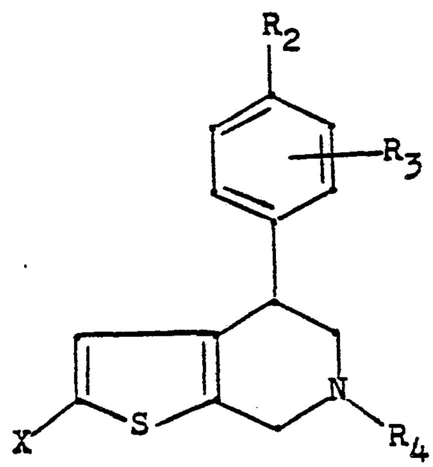

(II)

dans laquelle les radicaux $R_2$, $R_3$ et $R_4$ ont la signification indiquée plus haut et X représente un radical $NO_2$, $ClCH_2$—, $BrCH_2$— ou HOC—,

a) pour la préparation de produits finals, dans lesquels $R_1$ représente le groupe amino ou le groupe acétylamino, on réduit le groupe nitro d'un composé de départ de formule générale II dans laquelle X représente $NO_2$, au moyen d'agents de réduction appropriés et on acétyle de manière usuelle, si on le désire, le composé amino ainsi obtenu, ou en ce que

b) pour la préparation de produits finals substitués en position 2 par le radical

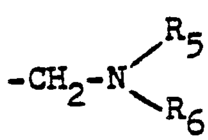

ou respectivement le groupe méthoxyméthyle, on fait réagir un composé de départ de formule générale II dans laquelle X représente un radical halogénométhyle, avec l'amine désirée ou respectivement avec l'ammoniac, ou un composé fournissant de l'ammoniac ou respectivement avec le méthylate de sodium, ou en ce que

c) pour la préparation de produits finals, dans lesquels $R_1$ représente un radical alcoylaminométhyle ou le groupe hydroxyméthyle, on fait réagir de manière en soi connue un composé de départ de formule générale II, dans laquelle X représente le radical formyle, soit avec une amine primaire et un agent de réduction approprié, soit avec le borohydrure de sodium, et en ce que l'on transforme un composé final ainsi obtenu de formule générale I de manière usuelle, en un sel d'addition d'acide physiologiquement inoffensif.

8. Procédé selon la revendication 7, caractérisé en ce qu'on transforme une 2-hydroxyméthyl-thiéno [2,3-c] pyridine de formule générale I ($R_1$ = $HOCH_2$—), au moyen de chlorure de thionyle et ensuite au moyen de méthylate de sodium en le composé 2-méthoxyméthylé correspondant.

9. Compositions pharmaceutiques contenant en tant que substance active un ou plusieurs composés de formule générale I en combinaison avec des adjuvants et/ou excipients usuels.